Europäisches Patentamt

European Patent Office

Office européen des brevets

(11) Veröffentlichungsnummer: **0 286 007**

**A2**

(12) # EUROPÄISCHE PATENTANMELDUNG

(21) Anmeldenummer: 88105222.9

(22) Anmeldetag: 31.03.88

(51) Int. Cl.⁴ **C12N 9/28 , C12N 9/44**

Der (Die) Mikroorganismus (Mikroorganismen) ist (sind) bei Deutsche Sammlung von Mikroorganismen unter der (den) Nummer(n) DSM 3389 und 2359 hinterlegt worden.

(30) Priorität: 09.04.87 DE 3712051

(43) Veröffentlichungstag der Anmeldung:
12.10.88 Patentblatt 88/41

(84) Benannte Vertragsstaaten:
AT BE CH DE ES FR GB IT LI NL

(71) Anmelder: **Kali-Chemie Aktiengesellschaft**
**Postfach 220 Hans-Böckler-Allee 20**
**D-3000 Hannover 1(DE)**

(72) Erfinder: **Antranikian, Garabed**
**Grisebachstrasse 8**
**D-3400 Göttingen(DE)**
Erfinder: **Gottschalk, Gerhard**
**Grisebachstrasse 8**
**D-3400 Göttingen(DE)**

(74) Vertreter: **Lauer, Dieter, Dr.**
**c/o Kali-Chemie AG Postfach 220**
**Hans-Böckler-Allee 20**
**D-3000 Hannover 1(DE)**

(54) **Thermostabile Amylasen und Pullulanasen aus 2 anaeroben Mikroorganismen.**

(57) Die Erfindung betrifft Amylasen und Pullulanasen, die aus Thermoanaerobacter finii DSM 3389 und Thermobacteroides acetoethylicus DSM 2359 gewonnen werden können. Die extrazellulären, thermostabilen Amylasen und Pullulanasen werden in kontinuierlichem Verfahren in hoher Konzentration aus den genannten thermophilen, anaeroben Mikroorganismen produziert. Diese Enzyme besitzen eine hohe Stabilität bei 60 °C und 70 °C in Abwesenheit von Metallionen und Substrat unter aeroben Bedingungen. Der pH-Wert und das Temperaturoptimum liegen im gleichen Bereich, so daß beide Enzyme gemeinsam zur Stärkehydrolyse eingesetzt werden können.

EP 0 286 007 A2

## Thermostabile Amylasen und Pullulanasen aus 2 anaeroben Mikroorganismen

Die Erfindung betrifft thermostabile Amylasen und Pullulanasen aus Thermoanaerobacter finii DSM 3389 und Thermobacteroides acetoethylicus DSM 2359.

Als Pullulanase wird ein Enzym definiert, das in der Lage ist, Pullulan zu hydrolysieren. Als Amylase wird ein Enzym definiert, das in der Lage ist, Stärke zu hydrolysieren.

Die Bildung von amylolytischen, d.h. Stärke abbauenden Enzymen ist bekannt. So wird nach WO-A-86.01 831 Clostridium thermohydrosulfuricum ATCC 33 223 ansatzweise auf einem Kulturmedium mit einem Gehalt an 0,5 % löslicher Stärke in Batchkultur kultiviert, wobei zwar Amylase, Pullulanase und Glucoamylase gebildet, jedoch nicht in das Kulturmedium ausgeschieden werden; vgl. auch Appl. Envir. Microbiol., 49 (1985) 1168-1173. Nach WO-A-86.01 832 wird Clostridium thermosulfurogenes ATCC 33 743 = DSM 2229 ansatzweise in einem Kulturmedium mit 0,5 % löslicher Stärke kultiviert, wobei Amylase, Pullulanase und Glucoamylase als amylolytische Enzyme gebildet werden, jedoch nur das zuerst genannte Enzym in das Kulturmedium austritt.

Aufgabe der Erfindung ist es, thermostabile amylolytische Enzyme bereitzustellen, die bei der Kultivierung von thermophilen, die genannten Enzyme bildenden Bakterien in hohem Maß in das Kulturmedium ausgeschieden werden und damit leicht isoliert werden können.

Thermoanaerobacter finii DSM 3389 und Thermobacteroides acetoethylicus DSM 2359 produzieren thermostabile Amylase und Pullulanase. Bei Stärke als Substrat werden in kontinuierlicher Kultur über 70 % beider Enzyme ins Medium ausgeschieden. Einzelheiten sind der folgenden Tabelle 1 zu entnehmen.

## Tabelle 1

Vergleich der Amylase- und Pullulanase-Gewinnung bei der chargenweisen und kontinuierlichen Kultur von Thermoanaerobacter finii DSM 3389 und Thermobacteroides acetoethylicus DSM 2359

| Organismus | chargenweise Kultur | | kontinuierliche Kultur | |
|---|---|---|---|---|
| | Amylase (U/l) | Pullulanase (U/l) | Amylase (U/l) | Pullulanase (U/l) |
| T. finii | 580 | 900 | 3000 | 3800 |
| T. aceto-ethylicus | 400 | 340 | 4100 | 3200 |

Bei chargenweisen Kultivierungen waren mehr als 80 % der amylolytischen Enzyme zellgebunden. Mehr als 70 % dieser Enzyme wurden in das Kulturmedium in Chemostaten abgegeben. T. finii DSM 3389 wurde bei einer Verdünnungsrate von 0,036/h und T. acetoethylicus DSM 2359 bei einer Verdünnungsrate von 0,030/h kultiviert. Der pH-Wert betrug 6,5.

Die in kontinuierlicher Kultur erreichten maximalen Aktivitäten beider Enzyme waren folgende:

1) Thermoanaerobacter finii DSM 3389

Pullulanase-Gesamtaktivität 3800 Einheiten/l (U/l)
Amylase-Gesamtaktivität 3000 Einheiten/l (U/l).
Die Bedingungen der optimalen Enzymproduktion im Fermenter waren
-pH 6,5
-1 % Stärke im Medium
-Durchflußrate 0,036/h
-Temperatur 65 °C

2) Thermobacteroides acetoethylicus DSM 2359

Pullulanase-Gesamtaktivität 3200 Einheiten/l (U/l)
Amylase-Gesamtaktivität 4100 Einheiten/l (U/l)
Die Bedingungen der optimalen Enzymproduktion im Fermenter waren
-pH 6,5
-1 % Stärke im Medium
-Durchflußrate 0,030/h
-Temperatur 65 °C

Charakterisierung der Enzyme

1) Thermoanaerobacter finii DSM 3389

Temperaturoptimum für Amylase und Pullulanase:

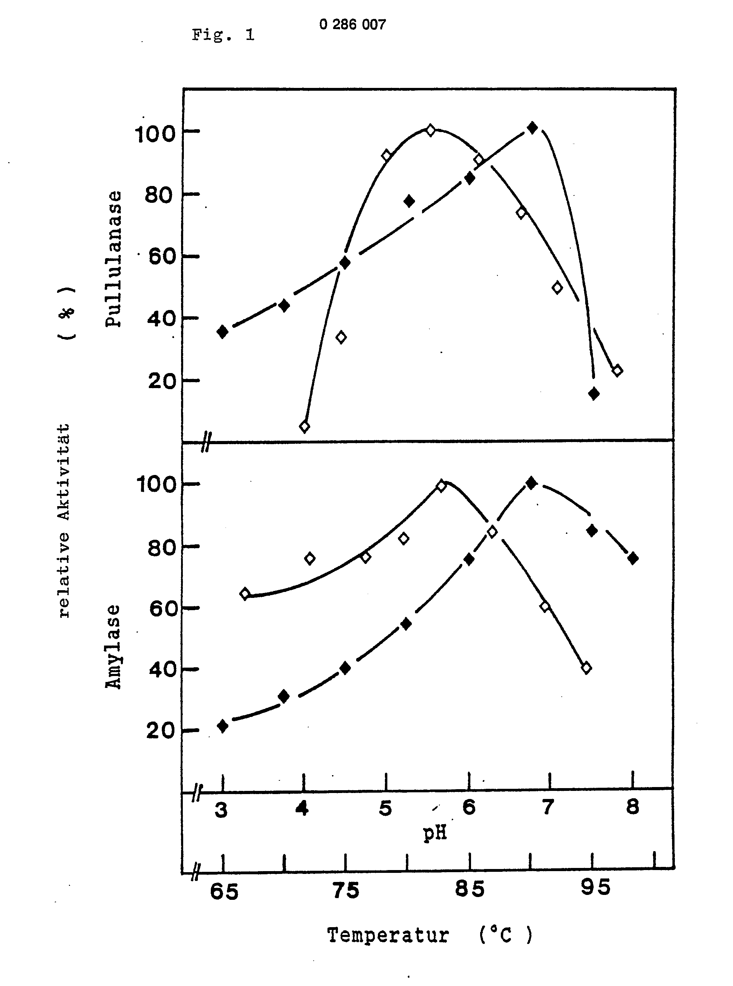

Beide Enzyme sind bei Temperaturen im Bereich von 50 bis 95 °C, insbesondere 75 bis 95 °C und vorzugsweise 80 bis 90 °C und besonders bevorzugt zwischen 85 und 90 °C aktiv. Siehe dazu Fig. 1.

pH-Optimum für Amylase und Pullulanase:

Beide Enzyme sind bei pH-Werten im Bereich von 4 bis 7, insbesondere 4,5 bis 6,5, vorzugsweise 5,0 bis 6,3 und besonders bevorzugt 5,0 bis 6,0 aktiv. Siehe dazu Fig. 1.

Beide Enzyme sind unter den gleichen Bedingungen (Temperatur und pH-Wert) katalytisch aktiv, so daß sie in einem Schritt gemeinsam zur Stärkehydrolyse eingesetzt werden können. Hierfür werden keine Metallionen benötigt. Man kann unter aeroben Bedingungen arbeiten.

Enzymaktivität in Gegenwart von Metallen:

Siehe dazu Tabelle 2. Die Zugabe von Ca-Ionen führt zur Aktivierung der Pullulanase (ca. 200 %).

Enzymaktivität in Gegenwart von Cyclodextrinen:

Siehe dazu Tabelle 2. Amylase und Pullulanase werden durch alpha-Cyclodextrin nicht gehemmt. In Gegenwart von beta-Cyclodextrin ist die Hemmung der Pullulanase deutlicher als die der Amylase.

Stabilität der Enzyme:

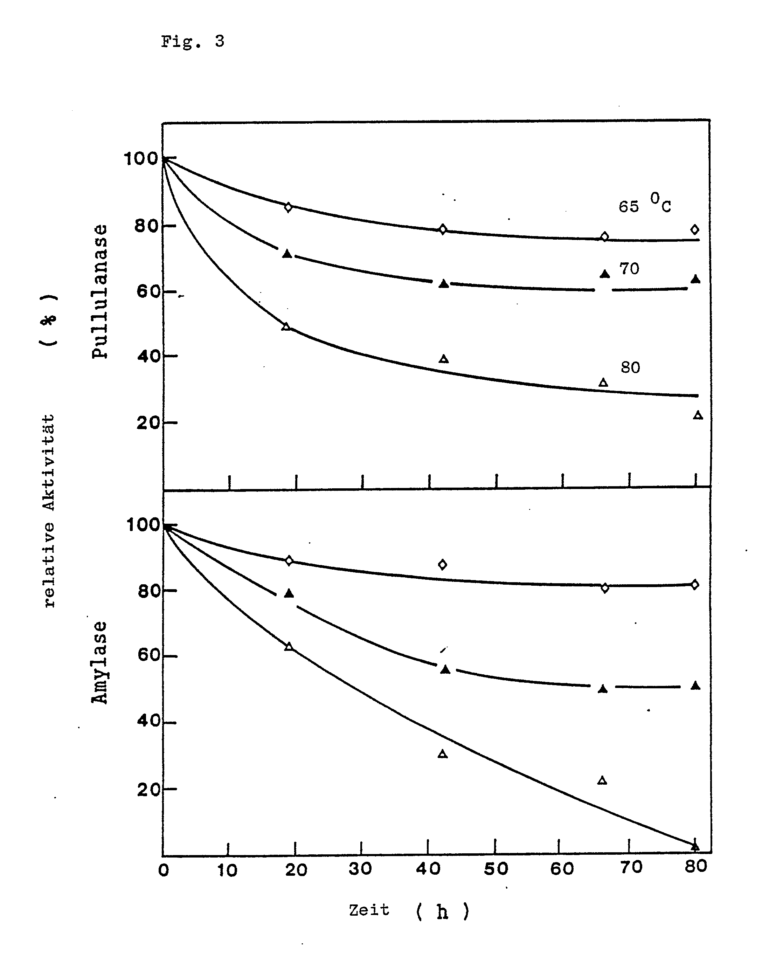

Beim pH-Wert von 5,5 wurde in Abwesenheit von Substrat und ohne Zusatz von Metallionen unter aeroben Bedingungen bei 60 °C und 65 °C nach 80 h kein Aktivitätsverlust festgestellt. Dies gilt für Amylase und Pullulanase. Die unter den gleichen Bedingungen gemessenen Stabilitäten bei 70 °C und 80 °C sind Fig. 3 zu entnehmen.

3

2) Thermobacteroides acetoethylicus DSM 2359

Temperaturoptimum für Amylase und Pullulanase:

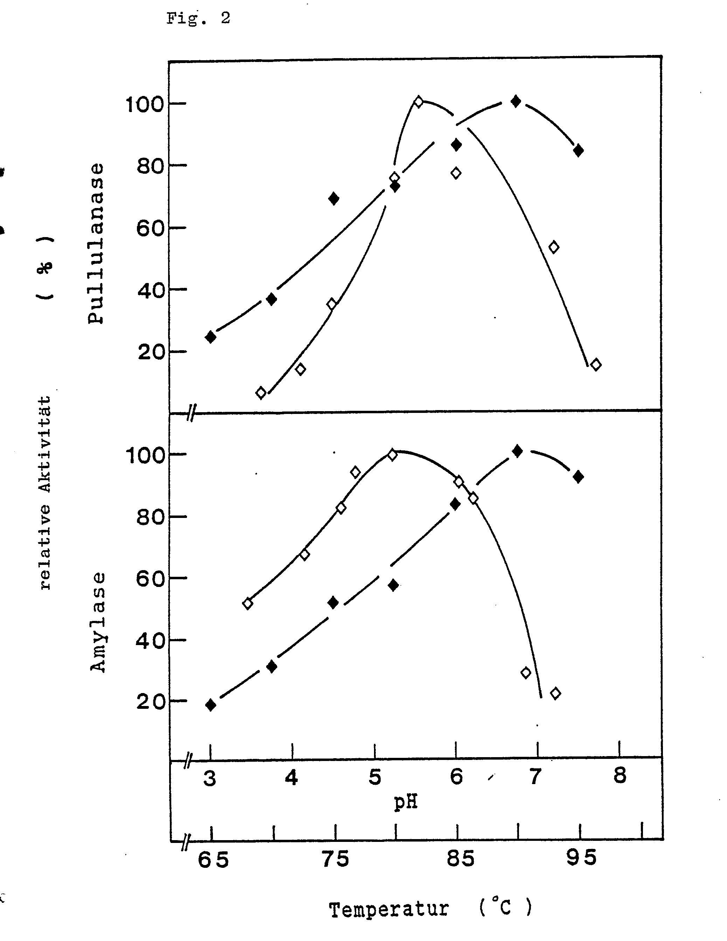

Beide Enzyme sind bei Temperaturen im Bereich von 65 bis 100 °C, insbesondere 75 bis 100 °C. vorzugsweise 80 bis 95 °C und besonders bevorzugt 85 bis 95 °C aktiv. Siehe dazu Fig. 2.

pH-Optimum für Amylase und Pullulanase:

Beide Enzyme sind bei pH-Werten im Bereich von 4,0 bis 7,0, insbesondere 4,5 bis 6,5 und vorzugsweise 5,0 bis 6,0 aktiv. Siehe dazu Fig. 2.

Beide Enzyme sind unter den gleichen Bedingungen katalytisch aktiv, so daß sie gemeinsam in einem Schritt zur Stärkehydrolyse eingesetzt werden können. Hierfür werden keine Metallionen benötigt. Man kann unter aeroben Bedingungen arbeiten.

Enzymaktivität in Gegenwart von Metallen:

Siehe hierzu Tabelle 2.

Enzymaktivität in Gegenwart von Cyclodextrinen:

Amylase und Pullulanase werden durch alpha-Cyclodextrin nicht gehemmt. beta-Cyclodextrin hemmt die Aktivität der Amylase leicht. Die Aktivität der Pullulanase wird gehemmt. Siehe dazu Tabelle 2.

Stabilität der Enzyme:

Beim pH-Wert von 5,5 wurde in Abwesenheit von Substrat und ohne Zusatz von Metallionen unter aeroben Bedingungen bei 60 °C nach 80 h kein Aktivitätsverlust festgestellt. Dies gilt für Amylase und Pullulanase. Nach 80 h Inkubation bei 70 °C wurden 60 ° der Amylase-und Pullulanase-Aktivitäten gemessen.

Fig. 1 zeigt den Einfluß von Temperatur und pH-Wert auf die Aktivität von Pullulanase und Amylase aus Thermoanaerobacter finii DSM 3389. Die Bestimmung des Temperaturoptimums der Enzymaktivitäten wurde in einem Natriumacetatpuffer (20 mmol/l) beim pH 5,5 vorgenommen. Die Bestimmung des pH-Optimums wurde mit dem genannten Puffer und Kaliumphosphatpuffer (20 mmol/l) und einer Inkubationstemperatur von 90 °C vorgenommen. Schwarze Rauten = Temperatur, weiße Rauten = pH-Wert.

Fig. 2 zeigt den Einfluß von Temperatur und pH-Wert auf die Aktivität von Pullulanase und Amylase aus Thermobacteroides acetoethylicus DSM 2359. Die Bedingungen und Symbole entsprechen denen der Figur 1.

Fig. 3 zeigt die thermische Stabilität von extrazellulärer Pullulanase und Amylase aus Thermoanaerobacter finii DSM 3389, wobei unter aeroben Bedingungen beim pH-Wert von 5,5 in Abwesenheit von Metallionen oder Substrat gearbeitet wurde. Die überstehende Flüssigkeit, welche die amylolytischen Enzyme enthielt, wurde steril abfiltriert und bei 65 °C (weiße Rauten), 70 °C (schwarze Rauten) und 80 °C (weiße Dreiecke) inkubiert.

Tabelle 2

Einfluß von zweiwertigen Kationen und Cyclodextrin auf die Aktivitäten von Amylasen und Pullulanasen aus T. finii DSM 3389 und T. acetoethylicus DSM 2359

| Bedingungen | T. finii | | T. acetoethylicus | |
|---|---|---|---|---|
| | Amylase (%) | Pullulanase (%) | Amylase (%) | Pullulanase (%) |
| keine Zugabe | 100 | 100 | 100 | 100 |
| Ca (mmol/l) | | | | |
| 0.4 | 85 | 210 | 130 | 100 |
| 1.6 | 90 | 200 | 40 | 90 |
| 4.6 | 120 | 190 | 20 | 90 |
| Zn (mmol/l) | | | | |
| 0.4 | 75 | 75 | 85 | 95 |
| 1.6 | 40 | 50 | 70 | 60 |
| 4.0 | 0 | 25 | 10 | 10 |
| Cu (mmol/l) | | | | |
| 0.4 | 95 | 90 | 100 | 100 |
| 1.6 | 0 | 30 | 40 | 45 |
| 4.0 | 0 | 0 | 5 | 10 |
| Mn (mmol/l) | | | | |
| 0.4 | 85 | 130 | 105 | 125 |
| 1.6 | 100 | 115 | 95 | 65 |
| 4.0 | 140 | 95 | 85 | 60 |
| EDTA (mmol/l) | 50 | 20 | 35 | 45 |
| beta-Cyclo-dextrin (mmol/l) | | | | |
| 1 | 105 | 15 | 60 | 20 |
| 2 | 105 | 5 | 45 | 10 |
| 5 | 95 | 5 | 40 | 10 |
| 7.5 | 100 | 5 | 50 | 5 |
| 10 | 100 | 0 | 40 | 0 |

Die Werte stellen jeweils den Mittelwert von 5 Messungen dar. Die Proben, welche die extrazellulären Enzyme aus T. finii und T. acetoethylicus enthielten, wurden in Natriumphosphatpuffer (20 mmol/l) beim pH 5,5 inkubiert. Es wurden Metallionen und Cyclodextrine in verschiedenen Konzentrationen zugegeben; die Umsetzung wurde bei 90 °C durchgeführt. Andere Metallionen (bis zu 4 mmol/l), wie Mg, Ni, Mo und Co, zeigten keine signifikante Wirkung. In Gegenwart von alpha-Cyclodextrinen (1 bis 10 mmol/l) wurde eine leichte Abnahme der Aktivitäten der amylolytischen Enzyme beider Bakterien gemessen; in Gegenwart von 10 mmol/l alpha-Cyclodextrin wurden noch 70 % Enzym ermittelt.

Die Gewinnung der erfindungsgemäßen Enzyme erfolgt gemäß dem in der europâischen Patentanmeldung 87 11 6625.2 beschriebenen Verfahren.

Danach wird die Exkretion von amylolytischen Enzymen (Amylasen und Pullulanasen) aus thermophilen, die genannten Enzyme bildenden anaeroben Bakterien in das Kulturmedium gefördert, wenn man die genannten Bakterien in einem Kohlenstoff-Quellen, Stickstoff-Quellen Mineralsalze und gegebenenfalls Vitamine enthaltenden Kulturmedium kontinuierlich kultiviert und als Kohlenstoff-Quelle ein höheres Saccharid in das Wachstum der Bakterien limitierenden Konzentrationen verwendet.

Der Ausdruck "höheres Saccharid" ist im vorliegenden Zusammenhang so zu verstehen, daß Monosaccharide ausgenommen sind. Beispiele für höhere Saccharide sind Polysaccharide, wie Stärke, Dextrin, Dextran oder Pullulan, und Oligosaccharide, wie Trisaccharide, z.B. Maltotriose, oder Disaccharide, z.B. Maltose, und deren Gemische.

Es ist dem Fachmann zuzumuten, die geeignete Konzentration des höheren Saccharids, den geeigneten pH-Wert und die geeignete Durchflußrate (Verdünnungsrate) zu ermitteln.

Lediglich zur Veranschaulichung sei angeführt, daß das Verfahren bei einer Konzentration an höherem Saccharid von 0,1 bis 3, vorzugsweise 0,2 bis 1,5 und insbesondere 0,5 bis 1,2 % (Gewicht/Volumen) durchgeführt werden kann.

Zur Veranschaulichung sei ferner ein pH-Wert von beispielsweise 4 bis 8, vorzugsweise 5,0 bis 7,5 und insbesondere 5,5 bis 7,0 genannt.

Zur Veranschaulichung sei schließlich eine Durchflußrate von beispielsweise 0,01 bis 0,4, insbesondere 0,02 bis 0,3, vorzugsweise 0,02 bis 0,2 und besonders bevorzugt von 0,03 bis 0,15 $h^{-1}$ genannt.

Hinsichtlich der Wahl einer geeigneten Temperatur wird sich der Fachman danach richten, daß das Temperatur-Minimum durch zu langsames Zellwachstum und das Temperatur-Maximum durch Proteindenaturierung und Absterben der Zellen vorgegeben werden.

Als Kulturmedium hat sich ein Medium bewährt, das neben dem höheren Saccharid die folgenden Bestandteile enthielt (% Gewicht/Volumen): 0,25 Trypton; 0,1 Hefe-Extrakt; 0,33 $KH_2PO_4$; 0,016 $MgCl_2$; 0,0012 $CoCl_2.6H_2O$; 0,05 Cystein.HCl. Pro l Medium wurde jeweils 1 ml einer Vitaminlösung und einer Spurenelementlösung zugegeben.

Die Kultivierung wurde in 2-l-oder 5-l-Fermentoren bei einem Kulturvolumen von 1 l bis 2 l bei 60 °C durchgeführt. Die Kulturen wurden durch einen kontinuierlichen Stickstoffstrom anaerob gehalten. Der Stickstoff wurde sterilisiert, indem man ihn durch ein sterilisiertes Baumwoll-Filter strömen ließ. Die Kulturen wurden mit 150 U/min gerührt. Der pH-Wert wurde mit einer Glaselektrode gemessen und bei dem vorgegebenen Wert gehalten, indem man automatisch 2 N KOH zugab. Die Isolierung der Enzyme erfolgte nach bekannten Techniken.

## Ansprüche

1. Amylolytische Enzyme, erhältlich durch kontinuierliches Kultivieren von thermophilen anaeroben Bakterien, ausgewählt aus der Gruppe Thermoanaerobacter finii DSM 3389 und Thermobacteroides acetoethylicus DSM 2359, in einem Kulturmedium, welches Kohlenstoffquellen, Stickstoffquellen, Mineralsalze und gegebenenfalls Vitamine enthält, wobei als Kohlenstoffquelle ein höheres Saccharid unter in bezug auf das Wachstum der Bakterien limitierenden Konzentrationen verwendet wird.

2. Amylase, erhältlich aus Thermoanaerobacter finii DSM 3389.

3. Amylase gemäß Anspruch 2 mit einer optimalen Aktivität bei Temperaturen zwischen 50 °C und 95 °C, insbesondere zwischen 75 °C und 95 °C, vorzugsweise zwischen 80 °C und 90 °C und besonders bevorzugt zwischen 85 °C und 90 °C und bei einem pH-Wert zwischen 4,5 und 6,5, vorzugsweise 5,0 und 6,3, insbesondere zwischen 5,0 und 6,0.

4. Pullulanase, erhältlich aus Thermoanaerobacter finii DSM 3389.

5. Pullulanase gemäß Anspruch 4 mit einer optimalen Aktivität bei Temperaturen zwischen 50 °C und 95 °C, insbesondere zwischen 75 °C und 95 °C, vorzugsweise zwischen 80 °C und 90 °C und besonders bevorzugt zwischen 85 °C und 90 °C und bei einem pH-Wert zwischen 4,5 und 6,5, vorzugsweise 5,0 und 6,3, insbesondere zwischen 5,0 und 6,0.

6. Amylase, erhätlich aus Thermobacteroides acetoethylicus DSM 2359.

7. Amylase gemäß Anspruch 6 mit einer optimalen Aktivität bei Temperaturen zwischen 65 °C und 100 °C, insbesondere zwischen 75 °C und 100 °C, vorzugsweise zwischen 80 °C und 95 °C und besonders bevorzugt zwischen 85 °C und 90 °C und bei einem pH-Wert zwischen 4,5 und 6,5, vorzugsweise 5,0 und 6,0.

8. Pullulanase, erhältlich aus Thermobacteroides acetoethylicus DSM 2359.

9. Pullulanase gemäß Anspruch 8 mit einer optimalen Aktivität bei Temperaturen zwischen 65 °C und 100 °C, insbesondere zwischen 75 °C und 100 °C. vorzugsweise zwischen 80 °C und 95 °C und besonders bevorzugt zwischen 85 °C und 90 °C und bei einem pH-Wert zwischen 4,5 und 6,5, vorzugsweise 5,0 und 6,0.

Fig. 1

0 286 007

Fig. 2

Fig. 3